# EUROPEAN PATENT APPLICATION

(11) **EP 4 154 886 A1**
(43) Date of publication of application: **29.03.2023**
(21) Application number: 21809693.1
(22) Date of filing: 12.05.2021
(51) Int. Cl.: A61K 31/496, A61K 45/00, A61P 21/00, C07D 401/04

(54) **CUG REPEAT SEQUENCE BINDING AGENT**

(30) Priority: 20.05.2020 JP 2020088499
(71) Applicant: OSAKA UNIVERSITY, Suita-shi Osaka 565-0871 (JP)
(72) Inventor: NAKATANI Kazuhiko, Suita-shi, Osaka 565-0871 (JP); MATSUMOTO Jun, Suita-shi, Osaka 565-0871 (JP); OKAMOTO Tatsumasa, Suita-shi, Osaka 565-0871 (JP); DOHNO Chikara, Suita-shi, Osaka 565-0871 (JP); SEIKE Asako, Suita-shi, Osaka 565-0871 (JP); NAKAMORI Masayuki, Suita-shi, Osaka 565-0871 (JP)
(74) Representative: Lederer & Keller Patentanwälte Partnerschaft mbB
(86) International application number: PCT/JP2021/018079
(87) International publication number: WO 2021/235293

(57) **Abstract**

Provided is a novel agent capable of binding to a CUG repeat sequence. The agent comprises a compound A having a binding response of 10 resonance units (RU) or more at 25 nM to a (CUG)₉ RNA immobilized at 401 RU as determined by surface plasmon resonance.

## Description

### TECHNICAL FIELD

The present invention relates to a novel agent capable of binding to a CUG repeat sequence, etc.

### BACKGROUND ART

Repeat expansion diseases or triplet repeat diseases are hereditary neurological diseases caused by abnormal expansion of trinucleotide repeat sequences in a gene.

For example, the repeat expansion disease myotonic dystrophy type 1 (DM1) is caused by abnormal expansion of CTG repeat sequences in the myotonic dystrophy protein kinase (DMPK) gene. Specific pathogenesis of DM1 is thought to involve RNAs with CUG repeats (CUG repeat RNAs) transcribed from the abnormally expanded CTG repeat sequences. CUG repeat RNAs bind to and aggregate with RNA-binding proteins and may lead to reduction in protein function.

Molecules that bind to CUG repeat RNAs are capable of dissociating RNA-binding proteins from aggregates of CUG repeat RNAs and the RNA binding proteins, and reportedly such molecules can serve as a molecular tool useful for studies on the treatment of DM1 (non-patent literatures 1 to 3).

### CITATION LIST

### NON-PATENT LITERATURE

Non-patent literature 1: Chem. Eur. J. 2016, 22, 14881-14889.
Non-patent literature 2: Bioorg. Med. Chem. Lett. 2016, 26, 3761-3764.
Non-patent literature 3: Chem. Eur. J. 2018, 24, 18115-18122.

### SUMMARY OF INVENTION

### TECHNICAL PROBLEM

An object of the present invention is to provide a novel agent capable of binding to a CUG repeat sequence.

Another object of the present invention is to provide a novel agent for treating and/or preventing a repeat expansion disease.

### SOLUTION TO PROBLEM

Molecules with therapeutic potential in the treatment of DM1 were reported previously as described above. However, there is no specific guidance for what types of compounds may lead to cure of DM1, and researchers have to create molecular designs for such compounds by repeated trial and error.

The inventors conducted extensive studies to solve the above problems and found that a compound can be experimentally determined to be able to bind to a CUG repeat sequence when the binding capacity of the compound to the CUG repeat sequence as determined by surface plasmon resonance (SPR) indicates a definite value in a specific metric or measure. The inventors also found that the metric is directly associated with the improvement of the splicing abnormality in repeat expansion diseases, such as DM1. The inventors also found that when the binding capacity of a compound is strong enough to indicate the specific value, the compound can be effective for the treatment of repeat expansion diseases. The inventors further found that the molecular design of a compound capable of improving the splicing abnormality in repeat expansion diseases, such as DM1, can be easily carried out using such a metric.

The inventors also found a novel compound containing a specific structural unit. The inventors further found that such a novel compound is effective for the treatment based on the above metric. The inventors made further studies and completed the present invention.

That is, the present invention relates to the following.
(1) An agent capable of binding to a CUG repeat sequence, the agent comprising a compound A having a binding response of 10 resonance units (RU) or more at 25 nM to a (CUG)₉ RNA immobilized at 401 RU as determined by surface plasmon resonance.
(2) An agent for treating and/or preventing a repeat expansion disease, the agent comprising a compound A having a binding response of 10 resonance units (RU) or more at 25 nM to a (CUG)₉ RNA immobilized at 401 RU as determined by surface plasmon resonance.
(3) The agent according to the above (1) or (2), wherein the compound A has a skeleton capable of forming a complementary hydrogen bond with a uracil.
(4) The agent according to any one of the above (1) to (3), wherein the compound A has a structure represented by the following formula (1): 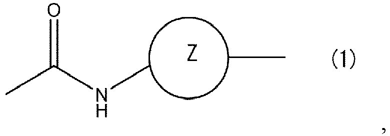 wherein Z represents an aromatic ring.
(5) A compound having one or more structural units represented by the following formula (1A): 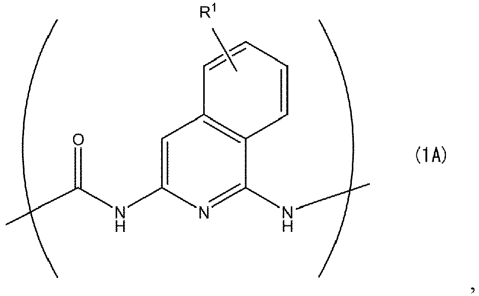 wherein R¹ represents a substituent.
(6) The compound according to the above (5), wherein R¹ in the formula (1A) is an aromatic ring group.
(7) The agent according to any one of the above (2) to (4), wherein the repeat expansion disease is caused by abnormal expansion of CTG repeats (CTG repeat diseases).
(8) The agent according to any one of the above (2) to (4) and (7), wherein the repeat expansion disease is myotonic dystrophy type 1.
(9) An administration method comprising administering, to an animal including humans, a compound having one or more structural units represented by the following Formula (1A): 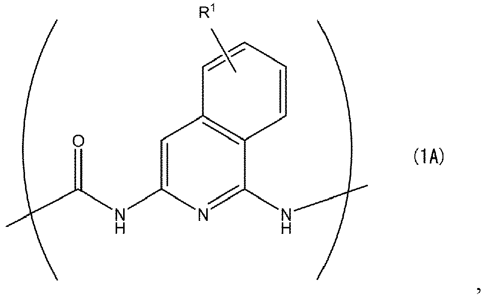 wherein R¹ represents a substituent.
(10) A method for treating and/or preventing a repeat expansion disease, the method comprising the administration according to the above (9).
(11) A method for screening for a compound A (a substance A), the method comprising identifying a compound A (a substance A) having a binding response of 10 resonance units (RU) or more at 25 nM to a (CUG)₉ RNA immobilized at 401 RU as determined by surface plasmon resonance, wherein the binding response is used as a metric.
(12) The method according to the above (11), wherein the compound A is an agent capable of binding to a CUG repeat sequence, or a compound or substance capable of binding to a CUG repeat sequence, or a compound or substance capable of effectively binding to a CUG repeat sequence.
(13) The method according to the above (12), wherein the compound A is an agent for treating and/or preventing a repeat expansion disease, or a compound or substance for treating and/or preventing a repeat expansion disease, or a compound or substance effective for treating and/or preventing a repeat expansion disease.
(14) Use of a compound A as an agent capable of binding to a CUG repeat sequence, wherein the compound A has a binding response of 10 resonance units (RU) or more at 25 nM to a (CUG)₉ RNA immobilized at 401 RU as determined by surface plasmon resonance.
(15) Use of a compound A as an agent for treating and/or preventing a repeat expansion disease, wherein the compound A has a binding response of 10 resonance units (RU) or more at 25 nM to a (CUG)₉ RNA immobilized at 401 RU as determined by surface plasmon resonance.
(16) A method comprising administering a compound A to an animal including humans and allowing the compound A to bind to a CUG repeat sequence, wherein the compound A has a binding response of 10 resonance units (RU) or more at 25 nM to a (CUG)₉ RNA immobilized at 401 RU as determined by surface plasmon resonance.
(17) A method for treating and/or preventing a repeat expansion disease, the method comprising administering, to an animal including humans, a compound A having a binding response of 10 resonance units (RU) or more at 25 nM to a (CUG)₉ RNA immobilized at 401 RU as determined by surface plasmon resonance.

### ADVANTAGEOUS EFFECTS OF INVENTION

The present invention provides a novel agent capable of binding to a CUG repeat sequence.

The agent can be used to improve the splicing abnormality in repeat expansion diseases, such as DM1 etc.

The present invention also provides a novel agent for treating and/or preventing a repeat expansion disease.

The agent is capable of binding to a CUG repeat sequence and can be used to treat and/or prevent a disease caused by CUG repeats transcribed from abnormally expanded CTG repeats, or a disease caused by abnormal expansion of CTG repeats (CTG repeat diseases).

The agent is capable of binding to a CUG repeat sequence and can be used to treat and/or prevent a disease caused by abnormal expansion of CAG repeats, which are a complementary strand of CTG repeats (CAG repeat diseases).

The present invention also provides a novel compound.

In an aspect, the present invention provides a novel agent for treating and/or preventing a repeat expansion disease.

### BRIEF DESCRIPTION OF DRAWINGS

Fig. 1 is a chart showing the results of SPR measurements in Example 1.
Fig. 2 is a chart showing the results of SPR measurements in Example 2.
Fig. 3 shows a schematic representation of alternative splicing of Atp2a1 (sarcoplasmic-reticulum Ca²⁺/ATPase gene) in normal subjects and DM1 patients.
Fig. 4 is a chart showing the evaluation results in Example 3.
Fig. 5 is a chart showing the evaluation results in Reference Example 1.
Fig. 6 shows a schematic representation of alternarive splicing of Clcn1 (muscle-specific chloride channel gene) in normal subjects and DM1 patients.
Fig. 7 is a chart showing the evaluation results in Example 4.
Fig. 8 is a chart showing the evaluation results in Reference Example 2.

### DESCRIPTION OF EMBODIMENTS

### Agent

The agent of the present invention comprises a compound A having a binding response of 10 resonance units (RU) or more at 25 nM to a (CUG)₉ RNA immobilized at 401 RU as determined by surface plasmon resonance (hereinafter the binding response measured under these conditions may be simply referred to as a "metric A").

Surface plasmon resonance (SPR) may be performed to determine the binding response of a compound of interest at 25 nM to a (CUG)₉ RNA immobilized at 401 RU. Alternatively, the binding response may be calculated from a value measured using the compound at a different concentration and/or a different amount of the immobilized RNA.

For example, the binding response in terms of resonance units when the concentration of a compound of interest is 25 nM and the amount of the immobilized (CUG)₉ RNA is 401 RU can be calculated by the formula: W × (401/Z) × (25/Y), wherein W is the measured resonance units (RU), Z is the amount of the immobilized (CUG)₉ RNA (RU), and Y is the concentration of the compound (nM).

Surface plasmon resonance can be measured by, for example, the method described below.

The device for measuring surface plasmon resonance may be, for example, BIAcore T200 (GE Healthcare).

The sensor chip for measuring surface plasmon resonance may be, for example, a streptavidin-coated sensor chip.

The buffer for measuring surface plasmon resonance may be, for example, HEPES buffer (e.g., HEPES-buffered saline, such as 10 mM HEPES buffer in 500 mM saline).

Among compounds having a metric A of 10 RU or more, a compound having a SPR response curve showing that binding of the compound to its ligand is maintained for some period of time and then the compound dissociates from the ligand, rather than raid dissociation, can serve as a compound A with more robust binding capacity to CUG repeats.

The agent of the present invention can serve as an agent capable of binding to a CUG repeat sequence, an agent for treating and/or preventing a repeat expansion disease, or other agents.

The agent of the present invention may comprise a single type or two or more types of compounds A.

### Compound A

A compound A typically has a metric A of 10 RU or more, and may preferably have a metric A of 11 RU or more (e.g., 12 RU or more, 13 RU or more, etc.).

The compound A with a metric A of 10 RU or more is capable of promoting normal splicing in Atp2a1 (sarcoplasmic-reticulum Ca²⁺/ATPase gene) or other genes and can be effective for treatment and/or prevention of repeat expansion diseases.

The metric A will be easily increased by various factors, such as skeletons capable of forming a complementary hydrogen bond with a uracil, aromatic rings (and their substituents), or the number of such skeletons or aromatic rings present in the compound, as described later. Accordingly, a compound having such a skeleton contributing to the increase of the metric A can suitably be employed as the compound A or as a skeleton that constitutes part of the compound A, or can suitably be used as an raw material or a precursor of the compound A, e.g., JM608.

The compound A may have, for example, a hydrogen bond group (e.g., a proton donor, a proton acceptor, etc.)

The hydrogen bond group may be a hydrogen bond donor group (or a proton donor) or a hydrogen bond acceptor group (or a proton acceptor).

The compound A may have one or more hydrogen bond groups.

The compound A may have a single type or two or more types of hydrogen bond groups.

The hydrogen bond donor group (or the proton donor) may be a group having an H atom attached to an atom with high electronegativity (e.g., an atom with a Pauling's electronegativity of 3 or more, such as an N atom or an O atom). The hydrogen bond donor group may be, for example, an -NH-group, an -NH₂ group, an -OH group, etc.

The number of the hydrogen bond donor groups in the compound A may be 1 or more, for example, 2 or more, and is preferably 3 or more, etc.

The hydrogen bond acceptor group (or the proton acceptor) may be a group having an atom with a lone pair (e.g., an N atom, an O atom, etc.), for example, a carbonyl group.

The number of the hydrogen bond acceptor groups in the compound A may be 1 or more and is preferably 2 or more, etc.

The compound A may preferably have a skeleton capable of forming a complementary hydrogen bond with a uracil. The number of the hydrogen bonds in the compound A may be 1 or more, and is preferably 2 or more, and more preferably 3 or more.

The compound A may be positively charged or become cationic when dissolved in water.

The molecule of the compound A may be basic itself.

The basic group(s) contained in the compound A may be, for example, an amino group etc.

The number of the basic group(s) contained in the compound A may be, for example, but is not limited to, 1 or more, e.g., 1 to 10, 1 to 7, 1 to 5, 1 to 3, etc., or may be 2 or more, e.g., 2 to 10, 2 to 7, 2 to 5, 2 to 3, etc.

The compound A may be a compound having an amide bond (-CONH-), a compound having an aromatic ring, or a compound containing an aromatic ring linked to an amide bond and having a structure represented by the following formula (1): 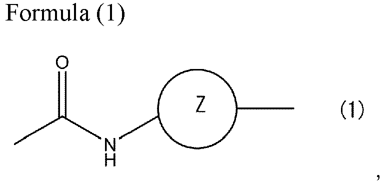 wherein Z represents an aromatic ring.

In the formula (1), Z represents an aromatic ring, which may be a hydrocarbon aromatic ring or a heterocyclic ring.

The number of carbon atoms in Z may be, for example, but is not limited to, 3 to 12, for example, 4 to 12, etc.

Z may be a monocyclic ring or a fused ring.

When Z is a heterocyclic ring, the heteroatom(s) may be, for example, but is not limited to, a nitrogen atom, an oxygen atom, a sulfur atom, etc. and is preferably a nitrogen atom, an oxygen atom, etc. The heterocyclic ring may have a single type or two or more types of heteroatoms.

The number of heteroatom(s) in the heterocyclic ring may be, for example, but is not limited to, 1 or more, for example, 1 to 5, 1 to 3, etc.

The position of the heteroatom(s) attached to the heterocyclic ring is not limited to a particular position.

Z may have a substituent.

Examples of the substituent include, for example, but are not limited to, alicyclic groups; aromatic ring groups; hydrocarbon groups, such as alkyl groups (e.g., C₁₋₅ alkyl groups, such as a methyl group and an ethyl group), alkenyl groups (e.g., C₂₋₅ alkenyl groups, such as an ethyl group and a propenyl group), alkynyl groups (e.g., C₂₋₅ alkynyl groups, such as an ethynyl group and a propynyl group); an amino group; a nitro group; etc. The substituent is preferably those having a ring structure, for example, an alicyclic group, an aromatic ring group, etc. When the substituent has a ring structure, the ring structure may be a hydrocarbon system or a heterocyclic ring.

When the substituent(s) on Z has a ring structure, the number of carbon atoms in the ring may be, for example, but is not limited to, 3 to 10, for example, 4 to 8, etc.

The number of the substituent(s) on Z may be one or two or more. The substituent(s) may be a single type or two or more types. The position of the substituent(s) on Z is not limited to a particular position, but the substituent(s) is preferably attached to a carbon atom.

Specific examples of the compound A having a structure represented by the formula (1) include a compound having one or more structural units represented by the following formula (1A): 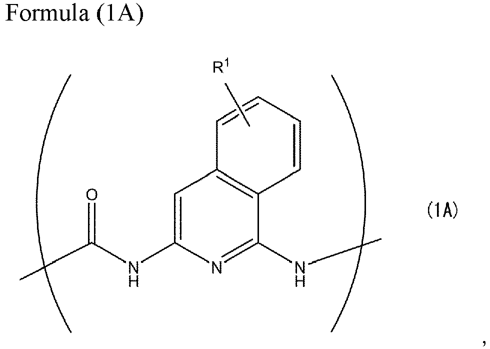 wherein R¹ represents a substituent.

R¹ in the formula (1A) is not limited to a particular substituent, and may be any one of those exemplified above for the substituents on Z.

R¹ is preferably an aromatic ring group, such as a hydrocarbon aromatic ring group and a heterocyclic aromatic ring group; an alkynyl group, such as a C₂₋₅ alkynyl group, such as an ethynyl group and a propynyl group; an amino group; a nitro group; etc. R¹ is particularly preferably an aromatic ring group. One reason of this is that when such a compound A having an aromatic ring group is bound to a repeat sequence (e.g., CUG repeats), the compound A has a stacking interaction with base pairs (e.g., G-C pairs in CUG repeats). Another reason is that such a compound A having an aromatic ring group may have a certain degree of stiffness and bulkiness and may easily be bound to a repeat sequence or an RNA having a repeat sequence.

Typical examples of the heterocyclic aromatic ring group include, for example, a pyridinyl group, a pyrimidinyl group, a pyrazinyl group, a pyrrolyl group, a furyl group, etc.

Typical examples of the hydrocarbon aromatic ring group include, for example, aryl groups, such as a phenyl group, a tolyl group, a xylyl group and a naphthyl group, etc.

R¹ may have a substituent (a).

The number of the substituent(s) (a) on R¹ may be one or two or more. The substituent(s) (a) may be a single type or two or more types.

The position of the substituent(s) (a) on R¹ is not limited to a particular position, but the substituent(s) is preferably attached to a carbon atom.

The substituent(s) (a) may be any one of those exemplified above for the substituents on Z. The substituent(s) (a) is preferably an alicyclic heterocyclic group, such as a group derived from an alicyclic amine. One reason of this is that such a compound A having an alicyclic heterocyclic group may have a certain degree of stiffness and bulkiness and may easily be bound to a repeat sequence or an RNA having a repeat sequence.

An exemplary substituent(s) (a) includes, for example, the following structures or groups:

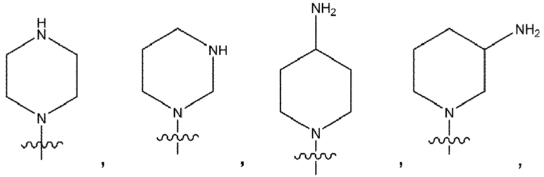

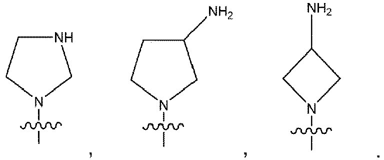

An exemplary R¹ includes, for example, the structures or groups shown below.

In the structures below, X represents a substituent (a), and n represents an integer of 0 or 1 or more.

When n is 2 or more, the substituents may be the same as or different from each other.

The maximum number of substituents (a) indicated by n may be selected depending on the type of group to be substituted (the type of aromatic ring group). For example, when the group to be substituted is pyridyl, the maximum number of substituents (a) may be 4. When the group to be substituted is furyl, the maximum number of substituents (a) may be 3.

The number of substituents (a) indicated by n may typically be 0 to 3, and is preferably 0 to 2, and further preferably 0 or 1, in particular, 1.

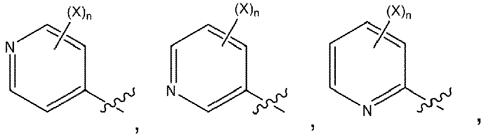

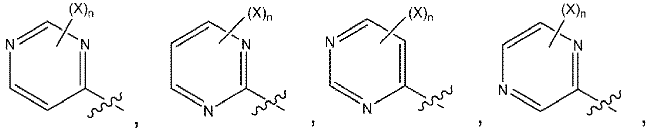

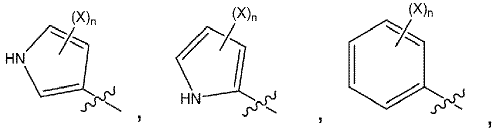

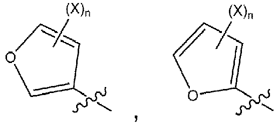

Among R¹ as exemplified above, the specific structures or groups shown below are preferred.

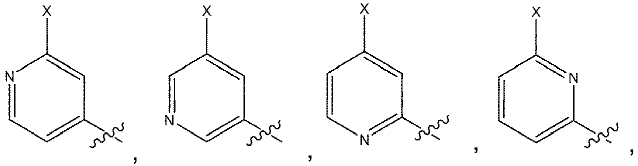

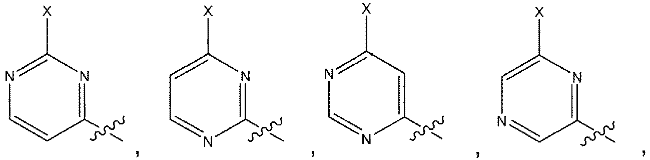

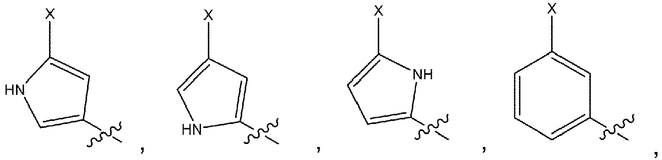

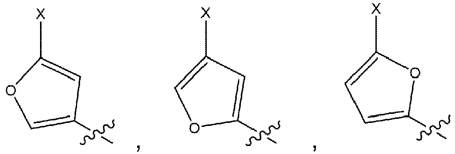

When the compound A has a structural unit represented by the formula (1A), the number of structural unit(s) represented by the formula (1A) in the compound A is 1 or more, but is preferably 2 or more (e.g., 2 to 5) or 3 or more, etc. to satisfy the metric A.

When the compound A has two or more structural units represented by the formula (1A), the structural units may be directly linked to each other, or may be liked via a linking group.

Examples of the linking group include, but are not limited to, heteroatom containing groups, such as an ether group (-O-), a thioether group (-S-), a carbonyl group (-CO-), a thiocarbonyl group (-CS-), an imino group (-NH-), an amide group (-NCO-), and a carbamoyl group (-NCOO-); hydrocarbon groups, such as saturated or unsaturated hydrocarbon groups, for example, alkylene or alkylidene groups (e.g., C₁₋₁₀ alkylene or alkylidene groups, such as methylene, ethylene, trimethylene, propylene and tetramethylene groups), cycloalkylene or cycloalkylidene groups (C₃₋₁₀ cycloalkylene or cycloalkylidene groups, such as cyclopropylene, cyclobutylene and cyclohexylene groups) and alkenylene groups (e.g., C₂₋₁₀ alkenylene groups, such as a vinylene group), and an arylene group (e.g., C₆₋₁₀ arylene groups, such as a phenylene group); a group formed of two or more of the same or different groups selected from these groups, such as a group formed of a heteroatom-containing group (one or two or more heteroatom-containing groups) and a hydrocarbon group (one or two or more hydrocarbon groups), for example, an oxyalkylene group, an alkylenedioxy group, an iminoalkylene group, etc.

The linking group may contain a hydrogen bond group, in particular, a group capable of forming a hydrogen bond with a uracil.

The molecular weight of the compound A is, for example, but not limited to, 200 or more, preferably 300 or more, more preferably 400 or more.

The compound A having a structural unit represented by the formula (1A) can be produced by a known organic synthesis method.

When the compound A has two or more structural units represented by the formula (1A), the structural units can be linked to each other by any known organic synthesis method.

The present invention also includes a novel compound.

The novel compound may be, for example, a compound having a structure represented by the formula (1), wherein Z is a fused heterocyclic ring, or a compound having one or more structural units represented by the formula (1A).

A compound having a single structural unit represented by the formula (1A) can be used as a raw material for producing a compound having two or more structural units represented by the formula (1A).

The compound used as a raw material does not need to have a metric A of 10 RU or more.

Surface plasmon resonance can be measured in any conventional manner.

The device for measuring surface plasmon resonance may be, for example, BIAcore T200 (GE Healthcare) as described in Examples below.

The sensor chip for measuring surface plasmon resonance may be, for example, a streptavidin-coated sensor chip (SA chip).

The buffer for measuring surface plasmon resonance may be, for example, HEPES buffer (e.g., HEPES-buffered saline, such as 10 mM HEPES buffer in 500 mM saline).

The activation buffer for measuring surface plasmon resonance may be, for example, a buffer containing 50 mM NaOH and 1 M NaCl.

The repeat expansion disease to be treated or prevented using the agent of the present invention include, for example, diseases caused by abnormal expansion of CTG repeats (CTG repeat diseases), such as myotonic dystrophy type 1, Huntington disease-like 2, spinocerebellar degeneration type 8, and Fuchs endothelial corneal dystrophy; diseases caused by abnormal expansion of CAG repeats (CAG repeat diseases), such as Huntington disease, spinal and bulbar muscular atrophy, and spinocerebellar degeneration type 2; and others.

The repeat expansion disease to be treated or prevented using the agent may be a single type or two or more types of diseases.

The dosage form of the agent of the present invention may be, for example, but not limited to, an injection, an eye drop, etc.

The agent of the present invention in the form of an injection or an eye drop may contain as needed, in addition to the compound A, various types of additives commonly used in the art, for example, a pH adjusting agent, a buffering agent, a stabilizer, an isotonic agent, a local anesthetic, etc.

The injection or eye drop can be prepared by adding various types of additives to the compound A in accordance with a conventional method.

Examples of the pH adjusting agent and the buffering agent include sodium citrate, sodium acetate, sodium phosphate, phosphate-buffered saline, etc.

Examples of the stabilizer include sodium pyrosulfite, ethylenediaminetetraacetic acid (EDTA), thioglycolic acid, thiolactic acid, etc.

Examples of the local anesthetic include procaine hydrochloride, lidocaine hydrochloride, etc.

Examples of the isotonic agent include sodium chloride, glucose, etc.

These additives may be used alone or in combination of two or more of them.

The amount of the compound A in the agent of the present invention is not limited to a particular amount, and may be determined as appropriate depending on the dosage range, the number of doses, or other factors.

The mode of administration of the agent of the present invention may be, for example, but is not limited to, intravenous, intramuscular, subcutaneous, intrathecal, and nasal administrations, etc.

The dosage range of the agent of the present invention is not limited to a particular dosage, and may be determined as appropriate depending on the dosage form, the mode of administration, the type of disease, the characteristics of the subject (e.g., the body weight, the age, the condition of the disease, use of other pharmaceuticals, etc.), or other factors.

The present invention is not limited to each of the embodiments as described above, and various modifications are possible. Embodiments obtainable by appropriately combining the technical means disclosed in the different embodiments of the present invention are also included in the present invention.

### EXAMPLES

The present invention will be described in more detail below with reference to Examples, but the present invention is not limited thereto.

### Synthetic Example 1

### Synthesis of compounds JM608 and JM642

Compounds JM608 and JM642 of the following structures were synthesized by the synthetic schemes below.

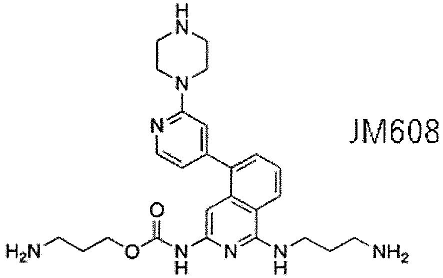

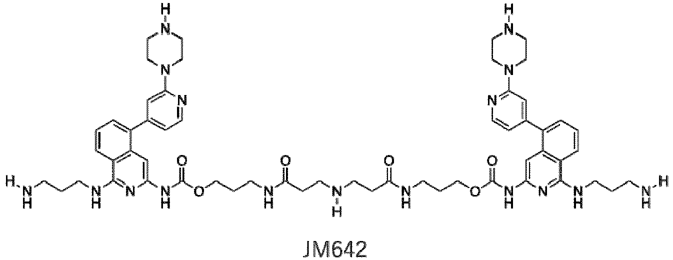

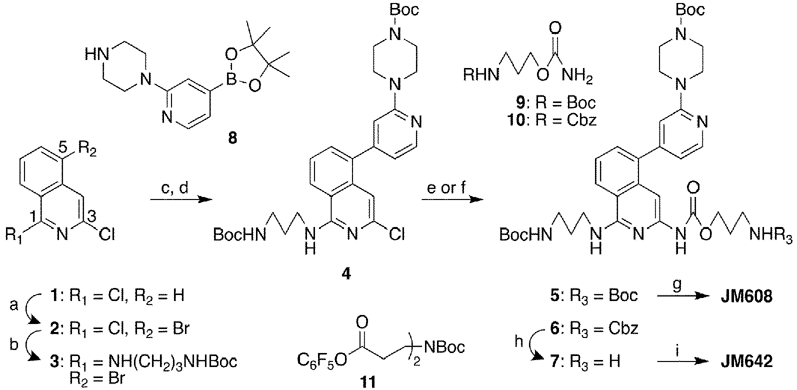

In this Synthetic Example, reagents were purchased from commercial suppliers and used without further purification.

HPLC was performed using Gilson 811C Dynamic Mixer (measurement wavelength: 254 nm, Cosmosil 5C₁₈-MS-II column (150 × 20 mm)) with a dual solvent system (0.1% AcOH/H₂O and MeCN).

¹H NMR and ¹³C NMR spectra were measured with ECS400 (JEOL), ECA600 (JEOL) and Avance III 700 (BRUKER).

ESI mass spectroscopy was performed using JEOL AccuTOF-T100N mass spectrometer.

The synthetic method of each compound following the synthetic schemes above is described below.

### Synthesis of 5-bromo-1,3-dichloroisoquinoline (compound 2) (step a)

1,3-dichloroisoquinoline (compound 1) (2.0 g, 10.1 mmol) and N-bromosuccinimide (2.3 g, 12.9 mmol) were mixed in super-dry acetonitrile (50 mL), then sulfuric acid (2 mL) was added dropwise and the mixture was stirred at room temperature for three days. The produced solid was separated by filtration and washed with hexane. The white solid was dried to give compound 2 (1.2 g, yield 44%).

The measurement results of compound 2 are as follows.
¹H NMR (600 MHz, CDCl₃): δ = 8.30 (d, J= 8.2 Hz, 1H), 8.03 (d, J= 7.6 Hz, 2H), 7.53 (m, 1H).
¹³C NMR (150 MHz, CDCl₃): δ = 151.6, 144.9, 138.4, 136.0, 129.0, 126.9, 126.5, 121.1, 119.5.
HRMS (ESI) m/z: calcd. for [C₉H₄⁷⁹Br³⁵Cl₂N + Na]⁺, 297.8796; found 297.8798.

### Synthesis of tert-butyl (3-((5-bromo-3-chloroisoquinolin-1-yl)amino)propyl)carbamate (compound 3) (step b)

Compound 2 (1.0 g, 3.6 mmol) was dissolved in 1,4-dioxane (8 mL), then diisopropylamine (1 mL) and N-Boc-1,3-propanediamine (3 mL) were added and the mixture was reflexed overnight. The resulting solution was neutralized with aqueous NH₄Cl solution and extracted with dichloromethane. The organic phase was dried with anhydrous magnesium sulfate and concentrated under reduced pressure. The residue was purified by silica gel column chromatography (eluted with 1% methanol/CHCl₃) to give compound 3 as a white solid (1.5 g, yield 54%).

The measurement results of compound 3 are as follows.
¹H NMR (600 MHz, CDCl₃): δ = 7.87 (d, J = 8.2 Hz, 1H), 7.83 (d, J = 7.6 Hz, 1H), 7.27 (t, J = 8.2 Hz, 1H), 7.21 (s, 1H), 6.79 (s, 1H), 5.05 (t, J = 5.8 Hz, 1H), 3.70 (q, J = 6.0 Hz, 2H), 3.25 (q, J = 5.5 Hz, 2H), 1.79 (quin, J = 5.8 Hz, 2H), 1.48 (s, 9H).
¹³C NMR (150 MHz CDCl₃): δ = 157.3, 155.9, 146.5, 138.1, 134.4, 126.0, 121.9, 121.1, 118.1, 106.9, 79.8, 37.7, 37.2, 29.9, 28.6.
HRMS (ESI) m/z: calcd. for [C₁₇H₂₉⁷⁹Br³⁵ClN₃O₂ + Na]⁺, 436.0398; found 436.0398.

### tert-Butyl 4-(4-(1-((3-((tert-butoxycarbonyl)amino)propyl)amino)-3-chloroisoquinolin-5-yl)pyridi n-2-yl)piperazine-l-carboxylate compound 4) (steps c and d)

Compound 3 (400 mg, 0.96 mmol), 1-(4-(4,4,5,5-tetramethyl-1,3,2-dioxaborolan-2-yl)pyridin-2-yl)piperazine (compound 8) (335 mg, 1.16 mmol), potassium carbonate (400 mg, 2.89 mmol) and Pd(PPh₃)₄ (111 mg, 96 µmol) in a solvent mixture of 1,4-dioxane (12 mL) and water (1 mL) were stirred at 80°C under argon atmosphere for 14 hours. The reaction mixture was cooled to room temperature, and Boc₂O (1.5 mL) was added and stirred for 1 hour. The reaction mixture was neutralized with aqueous NH₄Cl solution. The organic phase was dried with anhydrous MgSO₄ and concentrated under reduced pressure. The residue was purified by silica gel column chromatography (30% ethyl acetate/hexane) to give compound 4 as a pale yellow solid (493 mg, yield 86%).

The measurement results of compound 4 are as follows.
¹H NMR (600 MHz, CDCl₃): δ = 8.27 (d, J = 2.4 Hz, 11H), 7.93 (t, J = 4.7 Hz, 1H), 7.48 (d, J = 4.8 Hz, 2H), 6.85 (s, 1H), 6.71 (q, J = 2.1 Hz, 1H), 6.68 (t, J = 5.8 Hz, 1H), 6.65 (s, 1H), 5.14 (t, J = 6.2 Hz, 1H), 3.72 (q, J = 6.0 Hz, 2H), 3.58 (t, J = 4.8 Hz, 8H), 3.26 (q, J = 6.0 Hz, 2H), 1.79 (q, J = 5.5 Hz, 2H), 1.48 (s, 18H).
¹³C NMR (150 MHz, CDCl₃): δ = 159.6, 157.2, 155.9, 155.0, 149.3, 148.1, 145.5, 137.3, 136.7, 130.7, 125.3, 122.4, 117.0, 115.2, 108.1, 105.5, 80.1, 79.6, 45.2, 44.0, 42.9, 37.6, 37.2, 30.0, 28.6, 28.5.
HRMS (ESI) m/z: calcd. for [C₃₀H₄₂³⁵ClN₆O₄ + H]⁺, 597.2951; found 597.2955.

### Synthesis of tert-butyl 4-(4-(3-(((3-(tert-butoxycarbonyl)amino)propoxy)carbonyl)amino)-1-((3-((tert-butoxyc arbonyl)amino)propyl)amino)isoquinolin-5-yl)pyridin-2-yl)piperazine-1-carboxylate (compound 5) (step e)

A mixture of compound 4 (144 mg, 241 µmol), *tert*-butyl (3-(carbamoyloxy)propyl)carbamate (compound 9) (158 mg, 722 µmol), cesium carbonate (235 mg, 722 µmol) and XPhos Pd G3 (20 mg, 24 µmol) in super-dry 1,4-dioxane (9 mL) was reflexed under argon atmosphere for 15 hours. The reaction mixture was cooled to room temperature, diluted with ethyl acetate, filtered through a short plug of silica, and concentrated *in vacuo.* The residue was purified by silica gel column chromatography (40% ethyl acetate/hexane) to give compound 5 as a pale yellow solid (73.2 mg, yield 39%).

The measurement results of compound 5 are as follows.
¹H NMR (600 MHz, CDCl₃): δ = 8.28 (d, J = 5.6 Hz, 1H), 7.84 (d, J = 8.2 Hz, 1H), 7.50 (s, 1H), 7.46 (d, J = 7.3 Hz, 1H), 7.34 (t, J = 7.7 Hz, 1H), 7.12 (s, 1H), 6.78 (d, J = 4.7 Hz, 2H), 6.34 (s, 1H), 5.10 (s, 1H), 4.84 (s, 1H), 4.16 (t, J = 6.0 Hz, 2H), 3.65 (q, J = 6.2 Hz, 2H), 3.58 (d, J = 26.2 Hz, 8H), 3.26 (q, J = 5.7 Hz, 2H), 3.18 (s, 2H), 1.80 (m, 2H), 1.78 (m, 2H), 1.48 (s, 18H), 1.42 (s, 9H).
¹³C NMR (150 MHz, CDCl₃): δ = 159.5, 157.0, 156.1, 155.2, 155.0, 153.1, 149.9, 148.0, 145.4, 137.4, 137.1, 130.7, 123.4, 122.2, 116.1, 115.2, 108.6, 92.6, 80.0, 79.6, 79.3, 62.7, 45.2, 44.1, 42.9, 37.6, 37.5, 37.3, 29.9, 29.5, 28.6, 28.6, 28.6.
HRMS (ESI) m/z: calcd. for [C₄₀H₅₈N₈O₈ + H]⁺, 779.4450; found 779.4443.

### Synthesis of 3-aminopropyl (1-((3-aminopropyl)amino)-5-(2-(piperazin-1-yl)pyridin-4-yl)isoquinolin-3-yl)carbamat e (compound JM608) (step g)

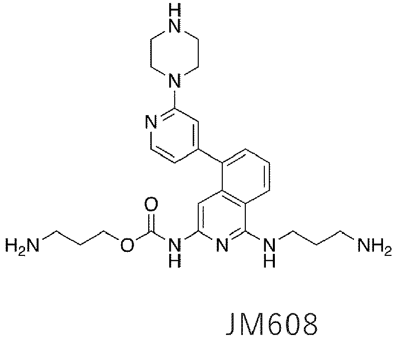

To a solution of compound 5 (8.5 mg, 10.8 µmol) in CHCl₃ (1 mL) was added ethyl acetate containing 4 M HCl (2 mL), and the reaction mixture was stirred at room temperature for 1 hour. The solvent was evaporated to dryness to give compound JM608 as a yellow solid (5.7 mg, yield 90%). The product was further purified by HPLC.

The measurement results of compound JM608 are as follows.
¹H NMR (600 MHz, D₂O): δ = 8.21 (d, J = 5.5 Hz, 1H), 7.96 (d, J = 8.2 Hz, 1H), 7.54 (d, J = 7.6 Hz, 1H), 7.44 (t, J = 7.9 Hz, 1H), 7.06 (s, 1H), 6.94 (s, 1H), 6.92 (d, J = 5.5 Hz, 1H), 4.20 (t, J = 5.8 Hz, 2H), 3.73 (t, J = 5.2 Hz, 4H), 3.67 (t, J = 6.5 Hz, 2H), 3.31 (t, J = 5.2 Hz, 4H), 3.09 (t, J = 7.2 Hz, 2H), 3.05 (t, J = 7.2 Hz, 2H), 1.92 (m, 2H), 1.91 (m, 2H)
¹³C NMR (150 MHz, D₂O): δ = 158.9, 156.1, 154.9, 150.8, 147.0, 144.5, 135.9, 135.9, 131.2, 124.2, 122.9, 116.6, 115.6, 109.9, 92.9, 62.7, 43.4, 43.1, 37.3, 36.9, 36.9, 27.1, 26.3.
HRMS (ESI) m/z: calcd. for [C₂₅H₃₄N₈O₂ + 2H]²⁺, 240.1475; found 240.1477.

### Synthesis of tert-butyl 4-(4-(3-(((3-(benzyloxy(carbonyl)amino)propoxy)carbonyl)amino)-1-((3-((tert-butoxyc arbonyl)amino)propyl)amino)isoquinolin-5-yl)pyridin-2-yl)piperazine-1-carboxylate (compound 6) (step f)

A mixture of compound 4 (500 mg, 837 µmol), benzyl (3-(carbamoyloxy)propyl)carbamate (compound 10) (317 mg, 1.26 mmol), cesium carbonate (818 mg, 2.51 mmol) and XPhos Pd G3 (71 mg, 84 µmol) in super-dry 1,4-dioxane (35 mL) was reflexed under argon atmosphere for 15 hours. The reaction mixture was cooled to room temperature, diluted with ethyl acetate, filtered through a short plug of silica, and concentrated *in vacuo.* The residue was purified by silica gel column chromatography (20-60% ethyl acetate/hexane) to give compound 6 as a pale yellow solid (356 mg, yield 52%).

The measurement results of compound 6 are as follows.
¹H NMR (600 MHz, CDCl₃): δ = 8.28 (d, J = 5.5 Hz, 1H), 7.84 (d, J = 7.6 Hz, 1H), 7.51 (s, 1H), 7.47 (d, J = 6.9 Hz, 1H), 7.32-7.36 (5H), 7.30 (t, J = 4.1 Hz, 1H), 7.10 (s, 1H), 6.78 (d, J = 4.1 Hz, 2H), 6.33 (s, 1H), 5.18 (s, 1H), 5.08 (s, 2H), 4.18 (t, J = 5.8 Hz, 2H), 3.65 (q, J = 5.5 Hz, 2H), 3.61 (d, J = 5.6 Hz, 4H), 3.56 (d, J = 5.5 Hz, 4H), 3.26 (d, J = 5.5 Hz, 4H), 1.84 (t, J = 6.2 Hz, 2H), 1.78 (t, J = 5.5 Hz, 2H), 1.48 (s, 9H), 1.48 (s, 9H).
¹³C NMR (150 MHz, CDCl₃): δ = 159.51, 156.97, 156.59, 155.22, 155.05, 153.14, 149.81, 148.06, 145.40, 137.40, 137.10, 136.69, 130.75, 128.61, 128.22, 128.19, 123.45, 122.15, 116.09, 115.13, 108.61, 92.67, 79.99, 79.67, 66.75, 62.65, 45.31, 43.98, 42.89, 37.95, 37.66, 37.37, 29.91, 29.49, 28.57.
HRMS (ESI) m/z: calcd. for [C₄₃H₅₆N₈O₈ + H]⁺, 813.4294; found 813.4298.

### Synthesis of tert-butyl 4-(4-(3-(((3-aminopropoxy)carbonyl)amino)-1-((3-((tert-butoxycarbonyl)amino)propyl) amino)isoquinolin-5-yl)pyridin-2-yl)piperazine-1-carboxylate compound 7) (step h)

Compound 6 (316 mg, 389 µmol) was dissolved in methanol (170 mL), and palladium carbon (Pd/C) (10% by mass) (60 mg) was added. The mixture was stirred under hydrogen gas at room temperature for 24 hours. The Pd/C was filtered through a short pad celite column, and the solvent was concentrated. The residue was purified on amino-coated silica gel eluted with 2% MeOH/CHCl₃ to give compound 7 as a pale yellow solid (219 mg, yield 74%).

The measurement results of compound 7 are as follows.
¹H NMR (600 MHz, CDCl₃: δ = 8.28 (d, J = 4.8 Hz, 1H), 7.83 (d, J = 8.2 Hz, 1H), 7.52 (s, 1H), 7.47 (d, J = 7.6 Hz, 1H), 7.35 (t, J = 7.9 Hz, 1H), 7.04 (s, 1H), 6.79 (d, J = 5.5 Hz, 2H), 6.28 (s, 1H), 5.10 (s, 1H), 4.21 (t, J = 6.2 Hz, 2H), 3.66 (q, J = 6.2 Hz, 2H), 3.61 (d, J = 5.9 Hz, 4H), 3.57 (d, J = 5.9 Hz, 4H), 3.26 (q, J = 5.7 Hz, 2H), 2.78 (t, J = 6.2 Hz, 2H), 1.80 (m, 2H), 1.79 (m, 2H), 1.49 (s, 9H), 1.48 (s, 9H).
¹³C NMR (176 MHz, CDCl₃): δ = 159.58, 156.95, 155.20, 155.03, 153.19, 149.85, 148.04, 145.46, 137.46, 137.13, 130.78, 123.43, 122.10, 116.04, 115.19, 108.57, 92.71, 79.99, 79.69, 63.03, 45.29, 44.11, 42.91, 38.96, 37.63, 37.32, 32.94, 29.95, 28.59. HRMS (ESI) m/z: calcd. for [C₃₅H₅₀N₈O₆ + H]⁺, 679.3926; found 679.3930.

### Synthesis of di-tert-butyl 4,4'-((((10-(tert-butoxycarbonyl)-7,13-dioxo-2,18-dioxa-6,10,14-triazanonadecanedioyl )bis(azanediyl))bis(1-((3-((tert-butoxycarbonyl)amino)propyl)amino)isoquinoline-3,5-di yl))bis(pyridine-4,2-diyl))bis(piperazine-1-carboxylate) (compound Boc-JM642) (step i)

Compound 7 (50 mg, 73.7 µmol) and bis(perfluorophenyl) 3,3'-((tert-butoxycarbonyl)azanediyl)dipropionate (compound 11) (19 mg, 32.5 µmol) were dissolved in CHCl₃ (1 mL), then triethylamine (47 µL, 338 µmol) was added, and the mixture was stirred at 50°C for 24 hours. The reaction mixture was cooled to room temperature, neutralized with aqueous NH₄Cl solution, and extracted with chloroform. The organic phase was dried with anhydrous magnesium sulfate and concentrated under reduced pressure. The residue was purified by silica gel column chromatography eluted with 1% MeOH/CHCl₃ to give compound Boc-JM642 as a pale yellow solid (51.9 mg, yield 89%).

The measurement results of compound Boc-JM642 are as follows.
¹H NMR (600 MHz, CDCl₃): δ = 8.25 (d, J = 5.5 Hz, 2H), 7.84 (d, *J=* 8.2 Hz, 2H), 7.50 (s, 2H), 7.44 (d, J = 6.9 Hz, 2H), 7.39 (s, 2H), 7.31 (t, J = 7.6 Hz, 2H), 6.77 (d, J = 5.5 Hz, 4H), 6.40 (s, 2H), 5.16 (t, J = 6.2 Hz, 2H), 4.11 (d, J = 5.5 Hz, 4H), 3.63 (m, 4H), 3.61 (m, 8H), 3.57 (m, 8H), 3.49 (m, 4H), 3.27 (d, J = 4.8 Hz, 4H), 3.22 (d, J = 6.2 Hz, 4H), 2.42 (s, 4H), 1.80 (t, J = 6.2 Hz, 4H), 1.74 (t, J = 5.5 Hz, 4H), 1.46 (s, 18H), 1.45 (s, 18H), 1.40 (s, 9H).
¹³C NMR (150 MHz, CDCl₃): δ = 159.47, 156.97, 155.86, 155.21, 155.03, 153.16, 149.83, 147.96, 145.50, 137.24, 137.01, 130.69, 123.35, 122.21, 116.06, 115.13, 108.61, 92.57, 80.30, 80.00, 79.56, 62.86, 45.33, 45.14, 42.85, 37.71, 37.42, 36.46, 36.07, 29.81, 28.94, 28.53, 28.48.
HRMS (ESI) m/z: calcd. for [C₈₁H₁₁₅N₁₇O₁₆ + 2H]²⁺, 791.9427; found 791.9434.

### Synthesis of ((3,3'-azanediylbis(propanoyl))bis(azanediyl))bis(propane-3,1-diyl)bis((1-((3-aminopro pyl)amino)-5-(2-(piperazin-1-yl)pyridin-4-yl)isoquinolin-3-yl)carbamate) (compound JM642)

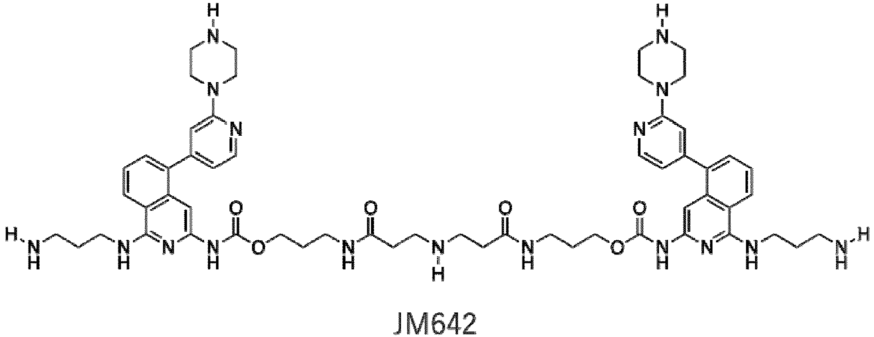

To a solution of compound Boc-JM642 (51.9 mg, 32.8 µmol) in chloroform (2 mL) was added ethyl acetate containing 4 M HCl (4 mL), and the reaction mixture was stirred at room temperature for 1 hour. The solvent was evaporated to dryness to give compound JM642 as a yellow solid (31.9 mg, yield 90%). The product was further purified by HPLC.

The measurement results of compound JM642 are as follows.
¹H NMR (600 MHz, D₂O): δ = 7.88 (d, J = 4.8 Hz, 2H), 7.49 (d, J = 8.2 Hz, 2H), 7.09 (d, J = 6.9 Hz, 2H), 6.97 (t, J = 7.6 Hz, 2H), 6.74 (s, 2H), 6.52 (d, J = 4.8 Hz, 2H), 6.37 (s, 2H), 3.88 (t, J = 5.8 Hz, 4H), 3.50 (t, J = 5.8 Hz, 4H), 3.40 (t, J = 4.8 Hz, 8H), 3.16 (t, J = 4.9 Hz, 8H), 3.10 (m, 4H), 3.06 (t, J = 5.1 Hz, 4H), 2.84 (t, J = 5.1 Hz, 4H), 2.80 (t, J = 6.9 Hz, 4H), 2.38 (t, J = 5.5 Hz, 4H), 1.86 (m, 4H), 1.62 (s, 4H).
¹³C NMR (176 MHz, D₂O): δ = 172.86, 158.28, 155.60, 154.72, 150.29, 146.64, 144.38, 135.39, 134.98, 131.10, 123.63, 122.62, 116.32, 115.24, 109.67, 91.98, 62.88, 43.51, 43.03, 42.97, 37.04, 36.67, 35.94, 32.01, 27.96, 27.17.
HRMS (ESI) m/z: calcd. for [C₅₇H₇₆N₁₆O₆ + 2H]²⁺, 541.8816; found 541.8821.

### Surface plasmon resonance (SPR) measurement

A streptavidin-coated sensor chip (SA chip, GE Healthcare) was washed with HBS-EP⁺ buffer (10 mM HEPES, pH 7.4, 0.15 M NaCl, 3 mM EDTA and 0.05% v/v Surfactant P20) for 6 minutes, and then activated with three consecutive 1-min injection of 30 µL of activation buffer (50 mM NaOH and 1 M NaCl).

5'-Biotinylated r(CUG)₉ (Thermo Fisher Scientific Inc.) was diluted to 0.1 µM with HEPES buffer (10 mM HEPES and 500 mM NaCl) and flowed onto the sensor chip until immobilized response units (RU) reaching around 400 RU. The amount of r(CUG)₉ immobilized on the surface of the sensor chip was at 401 RU.

Surface plasmon resonance was measured using BIAcore T200 SPR system (GE Healthcare).

r(CUG)₉ used for the SPR assay has the sequence as shown below.

**Table 1**

| | Sequence |
|---|---|
| r(CUG)₉ | 5'-biotin-TEG- CUGCUGCUGCUGCUGCUGCUGCUGCUG-3' |

### Example 1

For single-cycle kinetic (SCK) analysis, the surface of the sensor chip was conditioned by 120 sec exposure of continuous flow of HBS-EP⁺ buffer at a flow rate of 30 µL/min at 25°C. Compound JM608 was dissolved in HBS-EP⁺ buffer at a concentration of 0.063 µM, 0.125 µM, 0.25 µM, 0.50 µM and 1.0 µM, and the resulting solutions were sequentially injected on the sensor surface for 60 seconds each at a flow rate of 30 µL/min per cycle.

The obtained SPR response curves were analyzed using BIAcore T200 evaluation software (version 2.0).

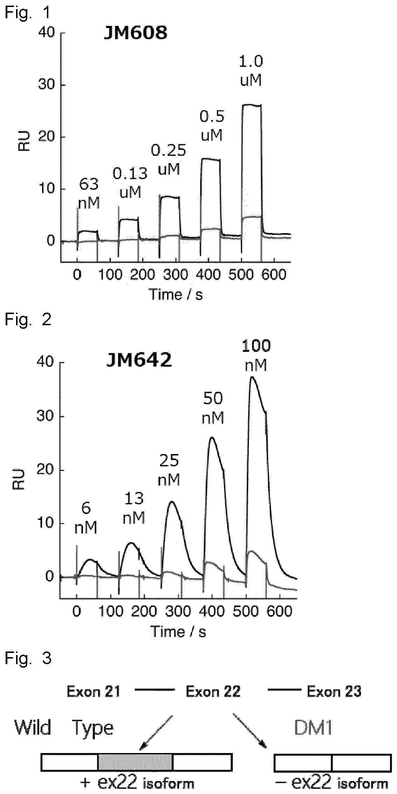

The results are shown in Fig. 1.

### Example 2

SPR measurement was performed in the same manner as in Example 1 except that compound JM642 was used in place of compound JM608 and that solutions of compound JM642 at concentrations of 6 nM, 13 nM, 25 nM, 50 nM and 100 nM were sequentially injected. The results are shown in Fig. 2.

The resonance units for the compounds at a concentration of 25 nM in Examples 1 and 2 are shown in Table 2 below.

The resonance unit for the compound at a concentration of 25 nM in Example 1 was calculated from the measured value (1.9 RU) for the compound at 63 nM using the formula: 1.9 RU × (25 nM/63 nM).

**Table 2**

| | Resonance unit (RU) at concentration of 25 nM |
|---|---|
| Example 1 | 0.8 |
| Example 2 | 14.3 |

As apparent from Figs. 1 and 2 and Table 2, the binding capacity to the CUG repeat sequence was observed in Examples 1 and 2.

### Studies on improvement in splicing abnormality in DM1 mouse model

### Example 3: Evaluation of Atp2a1 (sarcoplasmic reticulum Ca²⁺/ATPase gene)

Gender- and age-matched homozygous HSA^{LR} transgenic mice of line 20b (FVB inbred background) (Science 2000, 289, 1769-1772) (< 3 months old) were treated with JM642 at indicated doses (10 mg/kg/day and 20 mg/kg/day) for five days by daily intraperitoneal administration. After treatments, the rectus femoris (quadriceps) were obtained for splicing analysis. Total RNA extraction from the tissue, cDNA synthesis and polymerase chain reaction (PCR) were carried out according to Ann. Clin. Transl. Neurol. 2016, 3, 42-54. The PCR products were separated by agarose gel electrophoresis, and the gel was stained with GelRed (Biotium). The gel was imaged using a Typhoon laser fluorimager (GE Healthcare) and the gel bands were quantified using ImageQuant (GE Healthcare).

For normal mice and mice treated in the same manner as above except that JM642 was not administered, total RNA extraction, cDNA synthesis, PCR and electrophoresis of the PCR products were performed in the same manner as above.

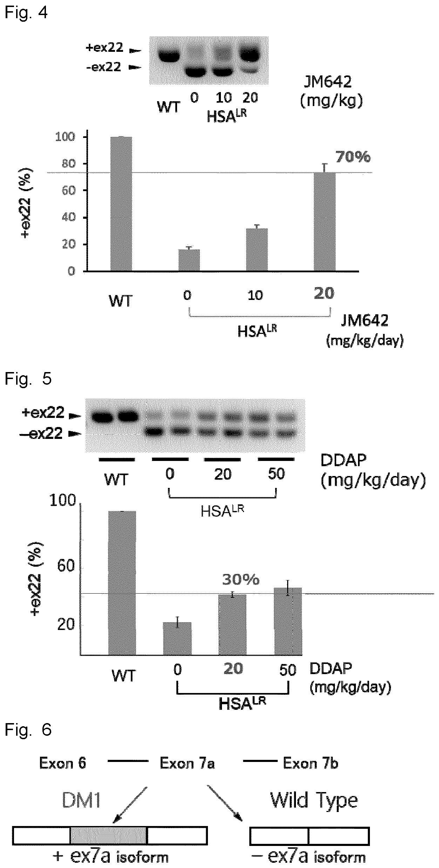

The results are shown in Fig. 4.

As shown in Fig. 3, in normal splicing of Atp2a1, Exon 22 is included or remains (see Wild Type in Fig. 3), whereas Exon 22 is excluded in DM1 patients.

As apparent from Fig. 4, the DM1 mouse model treated with compound JM642 demonstrated high inclusion rates for Exon 22 (ex22) in Atp2a1.

### Reference Example 1

Evaluation was performed in the same manner as in Example 3 except that the compound (DDAP) shown below synthesized in accordance with the method described in non-patent literature 3 was used in place of compound JM642. The results are shown in Fig. 5.

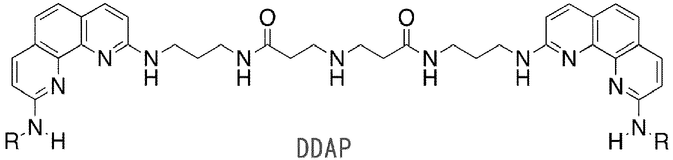

As shown in Figs. 4 and 5, for example, when each compound was administered at a dosage of 20 mg/kg/day, the inclusion rate of Exon 22 was 70% in Example 3 whereas the inclusion rate of Exon 22 was 30% in Reference Example 1, indicating that the splicing abnormality of DM1 in Example 3 was more improved than that in Reference Example 1.

### Example 4: Evaluation of Clcn1 (muscle-specific chloride channel gene)

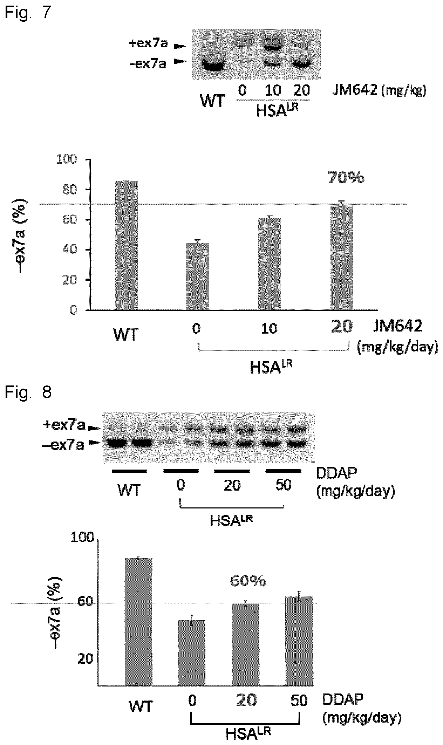

Evaluation was performed in the same manner as in Example 3 except that Clcn1 was studied in place of Atp2a1. The results are shown in Fig. 7.

As shown in Fig. 6, in normal splicing of Clcn1, Exon 7a is excluded (Wild Type), whereas Exon 7a is included in DM1 patients.

As apparent from Fig. 7, the DM1 mouse model treated with compound JM642 demonstrated high skipping rates for Exon 7a (ex7a) in Clcn1.

### Reference Example 2

Evaluation was performed in the same manner as in Example 4 except that the compound (DDAP) shown above synthesized in accordance with the method described in non-patent literature 3 was used in place of compound JM642. The results are shown in Fig. 8.

As shown in Figs. 7 and 8, for example, when each compound was administered at a dosage of 20 mg/kg/day, the skipping rate of Exon 7a was 70% in Example 4 whereas the skipping rate of Exon 7a was 60% in Reference Example 2, indicating that the splicing abnormality of DM1 in Example 4 was more improved than that in Reference Example 2.

### INDUSTRIAL APPLICABILITY

The present invention provides an agent useful as an agent for treating and/or preventing repeat expansion diseases, etc.

## Claims

1. An agent capable of binding to a CUG repeat sequence, the agent comprising a compound A having a binding response of 10 resonance units (RU) or more at 25 nM to a (CUG)₉ RNA immobilized at 401 RU as determined by surface plasmon resonance.

2. An agent for treating and/or preventing a repeat expansion disease, the agent comprising a compound A having a binding response of 10 resonance units (RU) or more at 25 nM to a (CUG)₉ RNA immobilized at 401 RU as determined by surface plasmon resonance.

3. The agent according to claim 1 or 2, wherein the compound A has a skeleton capable of forming a complementary hydrogen bond with a uracil.

4. The agent according to any one of claims 1 to 3, wherein the compound A has a structure represented by the following formula (1): 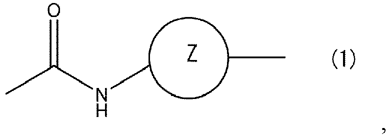 wherein Z represents an aromatic ring.

5. A compound having one or more structural units represented by the following formula (1A): 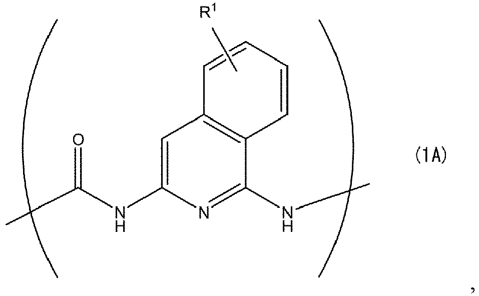 wherein R¹ represents a substituent.

6. The compound according to claim 5, wherein R¹ in the formula (1A) is an aromatic ring group.

7. The agent according to any one of claims 2 to 4, wherein the repeat expansion disease is caused by abnormal expansion of CTG repeats.

8. The agent according to any one of claims 2 to 4 and 7, wherein the repeat expansion disease is myotonic dystrophy type 1.

9. A method for screening for a compound A, the method comprising identifying a compound A having a binding response of 10 resonance units (RU) or more at 25 nM to a (CUG)₉ RNA immobilized at 401 RU as determined by surface plasmon resonance, wherein the binding response is used as a metric.

10. The method according to claim 9, wherein the compound A serves as an agent capable of binding to a CUG repeat sequence, an agent for treating a repeat expansion disease and/or an agent for preventing a repeat expansion disease.
